# EUROPEAN PATENT SPECIFICATION

(11) **EP 1 764 111 B1**
(45) Date of publication and mention of the grant of the patent: **09.12.2009**
(21) Application number: 06024500.8
(22) Date of filing: 28.10.2002
(51) Int. Cl.: A61K 47/32, A61K 9/00, A61K 31/167, A61P 15/02

(54) **Vaginally administered anti-dysrhythmic agents for treating uterine dysrhythimia**
Vaginal verabreichbare Zusammensetzung zur Behandlung von uteriner Dysrhythmie
Composition administrable par voie vaginale pour le traitement de la dysrythmie utérine

(30) Priority: 29.10.2001 US 330684 P; 24.10.2002 US 278912
(43) Date of publication of application: 21.03.2007
(62) Divisional of application: 02785326.6
(73) Proprietor: Columbia Laboratories (Bermuda) Limited, Pembroke HM08 (BM)
(72) Inventor: Levine, Howard, L., Oceanside NY 11572 (US); Bologna, William, J., 75017 Paris (FR); De Ziegler, Dominique, 1293 Geneva (CH)
(74) Representative: Keen, Celia Mary

(56) References cited:
- WO-A-00/10536
- WO-A-91/06290
- WO-A-95/07699
- WO-A-96/10989
- WO-A-98/56323
- WO-A-99/13862
- WO-A-99/15210
- US-A- 4 615 697
- US-A- 5 472 704
- US-A- 5 989 535

## Description

### FIELD OF THE INVENTION

This invention relates to a pharmaceutical composition for reducing or relieving uterine dysrhythmia. .The composition focuses on local topical use of certain anti-dysrhythmic treating agents for absorption into local tissue to prevent or treat the underlying abnormal or undesireable muscle contractions that are causing the pain or discomfort rather than merely relieving or masking the resulting pain or discomfort without affecting the cause.

### BACKGROUND OF THE INVENTION

Pelvic pain may be intermittent or recurrent, or it may be constant and severe, but it is frequently associated with uterine dysrhythmia abnormal, disordered, or disturbed contractions of the uterus. Pelvic pain is often experienced during menses, as painful menstruation, or dysmenorrhea. Women with chronic pelvic pain associated with menstruation frequently spend one day each month in bed and also may have an additional day each month of reduced activity because of the severity of the pain. Pelvic pain may also be caused by pelvic infections, and diseases of the urinary tract or bowel.

Infertility also may be associated with uterine dysrhythmic conditions, including dysmeaorrhea. *See*, *e.g.,* U.S. Patent Application Ser. No. 10/089,796. Uterine dysrhythmias may affect the rapid transport of sperm, thus affecting fertility. Contractility along the female tract (uterus and fallopian tubes) appears to be the primary motor assuring rapid transport of sperm from the cervical area to the distal end of the tubes, where fertilization takes place. Retrograde uterine contractility appears to impede this normal transport mechanism.

Chronic pelvic pain is common in women in the reproductive age group. It causes disability and distress, and results in significant costs to health services. Overall, a woman has about a 5% risk of having chronic pelvic pain for some period of time in her lifetime. In patients with a previous diagnosis of pelvic inflammatory disease this risk is increased fourfold to approximately 20%. Recent epidemiologic data from the United States showed that 14.7% of women in their reproductive ages reported chronic pelvic pain. A total of 15% of these women with chronic pelvic pain reported time lost from work and 45% reported reduced work productivity. In the United States 10% of outpatient gynecologic consultations are for chronic pelvic pain and 40% of laparoscopies are done for chronic pelvic pain.

The pathogenesis of chronic pelvic pain is poorly understood. Often, investigation by laparoscopy may reveal endometriosis, mild to moderate, or it may reveal no obvious cause for pain. There are several possible explanations for chronic pelvic pain including undetected irritable bowel syndrome, the vascular hypothesis where pain is thought to arise from dilated pelvic veins in which blood flow is markedly reduced and altered spinal cord and brain processing of stimuli in women with chronic pelvic pain. As the pathophysiology of chronic pelvic pain is not well understood, its treatment is often unsatisfactory and limited to symptom relief. Currently, the main approaches to treatment include symptomatic treatment of pain with medication, surgery, or possibly psychotherapy and counseling.

Very little is known about effective pharmacologic treatment for chronic pelvic pain, despite the fact that it is a very common chronic pain syndrome. Several different pharmacologic classes of medications have been used to alleviate the symptomatic pain and discomfort, rather than treat or prevent the underlying cause, in patients with chronic pain syndromes: nonsteroidal anti-inflammatory drugs, anticonvulsants, local anesthetics, and opioids. Very few studies have focused on the actual treatment or prevention of the underlying cause -- uterine dyskinetic contractions - in order to treat or prevent chronic pelvic pain.

Dysmenorrhea is associated with pain typically related to the menstrual cycle and can be primary or secondary. Most women experience primary dysmenorrhea at some time during their life. The pain is cramping or sharp and lasts the first few days of the menstrual period. It may radiate to the back, thighs, or deep pelvis. Occasionally, nausea or vomiting occurs. Secondary dysmenorrhea may be due to endometriosis or cervical stenosis or, if associated with heavy menstrual flow, to fibroids, adenomyosis, or large endometrial polyps.

In order to provide local or regional blockade for extended periods, clinicians currently use local anesthetics administered through a catheter or syringe to a site where the pain is to be blocked. This requires repeated administration where the pain is to be blocked over a period of greater than one day, either as a bolus or through an indwelling catheter connected to an infusion pump. These methods have the disadvantage of potentially causing irreversible damage to nerves or surrounding tissues due to fluctuations in concentration and high levels of anesthetic. In addition, anesthetic administered by these methods are generally neither confined to the target area, nor delivered in a linear, continuous manner. In all cases, analgesia rarely lasts for longer than six to twelve hours, more typically four to six hours. In the case of a pump, the infusion lines are difficult to position and secure, the patient has limited, encumbered mobility and, when the patient is a small child or mentally impaired, may accidentally disengage the pump.

U.S. Patent No. 5,700,485 discloses a method and device for administering a local anesthetic combined with a biodegradable polymer incorporated into microspheres. Prolonged release of the anesthetic is obtained by administration with glucocorticoid.

Because high systemic anesthetic concentration can cause irritation or burning to the vagina, as well as other detrimental side effects, there is a need to keep systemic circulation of the anesthesia low. Thus, there is a need for a formulation in which local anesthetics would diffuse preferentially into the cervix for a prolonged period of time to ensure sufficient anesthesia for treating pelvic pain due to dysrhythmic conditions, while keeping systemic circulation low.

. Similarly, high systemic levels of other anti-dysrhythmic treating agents may lead to adverse side effects, some of which may be severe. Many classic anti-arrhythmic (and other anti-dysrhydmic) agents themselves have the ability to cause coronary arrhythmia. Other detrimental side effects include without limitation nausea, blurred or yellow vision, precipitation of glaucoma, constipation, seizures, tremor, bone marrow aplasia, pulmonary fibrosis, hypotension, reduction of exercise heart rate, diarrhea and diarrhea-induced hypokalemia, and immunological reactions such as thrombocytopenia, hepatitis, or bone marrow depression. Thus, use of an anti-dysrhythmic agent to treat or prevent uterine dysrhythmia must carefully avoid systemic levels that could prompt coronary problems or other adverse side effects.

Accordingly, there is a need for a formulation that would locally and preferentially deliver anti-dysrhythmic treating agents to treat or prevent pelvic pain due to dysrhythmia, or to treat or improve infertility associated with dysrhythmia. The formulation should avoid blood levels of the treating agent high enough to cause detrimental side effects, while attaining sufficient local tissue levels of the treating agent to provide the desired therapeutic anti-dysrhythmic effect.

### SUMMARY OF THE INVENTION

The invention provides a pharmaceutical vaginal composition for reducing or relieving uterine dysrhythmia comprising an anti-dysrhythmic treating agent at a concentration of from 1% to 12.5% by weight and a pharmaceutically acceptable bioadhesive carrier that releases the treating agent over an extended period of time after administration, wherein the anti-dysrhythmic treating agent is selected from prilocaine, mepivacaine, bupivacaine, ropivacaine, etidocaine, mexiletine, moricizine, propafenone, flecainide and procainamide.

The invention also provides a use of an anti-dysrhythmic treating agent and a pharmaceutically acceptable bioadhesive carrier that releases the treating agent over an extended period of time after administration, in the manufacture of a medicament for vaginal administration in reducing or relieving uterine dysrhythmia, wherein the anti-dysrhythmic treating agent is present at a concentration of from 1% to 12.5% by weight and is selected from prilocaine, mepivacaine, bupivacaine, ropivacaine, etidocaine, mexiletine, moricizine, propafenone, flecainide and procainamide.

### DETAILED DESCRIPTION OF THE INVENTION

The present invention relates to a pharmaceutical composition that includes an effective amount of an anti-dysrhythmic treating agent, intended to reduce or relieve uterine dysrhythmia by normalizing propagation of the nerve impulses and/or nerve impulses or cell to cell communication (i.e., faster, slower, or more consistent) causing the abnormal or undesirable contractions, together with a pharmaceutically acceptable bioadhesive carrier. The particular anti-dysrhythmic treating agents used in the invention are either local anesthetics or classic "antiarrhythmics" normally associated with use for treating coronary dysrhythmias.

Local anesthetics are generally defined as a drug which may be used to provide local numbness or pain relief, by preventing the propagation of nerve impulses that relay or report the sensation of pain. The treating agents used in the present invention which are local anesthetics are prilocaine, mepivacaine, bupivacaine, ropivacaine and etidocaine.

The treating agents used in the present invention which are classic antiarrhythmics, generally used for treating or preventing coronary arrhythmias, are mexiletine, moricizine, propafenone, flecainide and procainamide,

The bioadhesive carrier includes a bioadhesive, water-swellable, water-insoluble, cross-linked polycarboxylic, polymer. In one embodiment the polymers comprises polycarbophil. A prefered carrier, which may be in a gel formulation contains a polycarbophil base designed to give controlled, extended release of the treating agent through the vaginal mucosa. Similar formulations for administration of different treating agents for other purposes are described in U.S. Patent Nos. 5,543,150 and 6,126,959,

U.S. Patent No. 5,543,150 discloses and claims use of similar extended-release vaginal formulations with progesterone to provide a FIRST UTERINE PASS EFFECT: directed local delivery of the progesterone to effect secretory transformation of the endometrium while maintaining very low blood serum levels of progesterone. Similarly, U.S. Patent No. 6,126,959 discloses and claims use and composition of other similar extended release formulations for vaginal delivery of treating agents to effect local efficacy without also causing detrimental blood levels of the treating agent.

The composition of the invention comprises from 1% to 12.5% concentrations of the treating agent.

The composition of the invention is to be applied vaginally, and may be formulated as any appropriate vaginal composition, such as, without limitation, a gel or cream, or even as a gelifying tablet for administration. When administered, the composition diffuses through the vaginal mucosal into the target tissue. Relief from pain is provided by treatment or prevention of the cause or source of the pain, e.g., increased or dysrhythmic contractility.

The treating agents in the instant compositions diffuse in high concentrations into the myometrium to alter dysfunctional uterine contractility for control of pain associated therewith. Systemic circulation of the treating agent remains at a low level, enabling the treatment to avoid adverse systemic side effects. Depending on both the treating agent and the formulation, which can be modified to extend or shorted the duration of release of the treating agent, the release and efficacy of the treating agent may easily last for at least about 48 hours or more.

The specific drug delivery formulation chosen includes a cross-linked polycarboxylic acid polymer formulation, generally described in U.S. Patent No. 4,615,697 . In general, at least about 80% of the monomers of the polymer in such a formulation should contain at least one carboxyl functionality. The cross-linldag agent should be present at such an amount as to provide enough bioadhesion to allow the system to remain attached to the target epithelial surfaces for a sufficient time to allow the desired dosing to take place. Of course, subject to the limitation that the treating agent is present at a concentration of from 1% to 12.5% by weight, higher does can be formulated readily by one of skill in the art to be released more slowly over a longer period of time; the key factor is the amount of treating agent administered per unit time, while the concentration of the formulation can be varied inversely with the amount of formulation per unit dosage, or varied directly with the duration of release of the treating agent. In other words, a higher concentration of treating agent in the formulation can be delivered more slowly, and/or in a smaller dose of the formulation, to achieve the same overall rate of delivery of the treating agent.

For vaginal administration, the formulation preferably remains attached to the epithelial surfaces for a period of about 24 to 48 hours. Such results may be measured clinically over various periods of time, by testing samples from the vagina for pH reduction due to the continued presence of the polymer. This level of bioadhesion is generally attained when the cross-linking agent is present at about 0.1 to 6 weight percent of the polymer, preferably about 1 to 2 weight percent. Bioadhesion can also be measured using commercially available surface tensiometers utilized to measure adhesive strength.

The polymer formulation can be adjusted to control the release rate of the treating agent by varying the amount of cross-linking agent in the polymer. Suitable cross-linking agents include divinyl glycol, divinylbenzene, N,N-diallylacrylamide, 3,4-dihydroxy-1,5-hexadiene, 2,5-dimethyl-1,5-hexadiene, and similar agents.

A preferred polymer for use in such a formulation is Polycarbophil, U. S.P., which is commercially available from Noveon, Inc., of Cleveland, Ohio under the trade name NOVEON®-AA1. Polycarbophil is a polyacrylic acid cross-linked with divinyl glycol

Other useful bioadhesive polymers that may be used in such a drug delivery system formulation are mentioned in the '697 patent. For example, these include polyacrylic acid polymers cross-linked with 3,4-dihydroxy-1,5-hexadiene, and polymethacrylic acid polymers cross-linked with divinyl benzene.

Typically, these polymers would not be used in their salt form, because this would decrease their bioadhesive capability. Divalent salts, such as calcium salts, cause the greatest decrease in bioadhesion. Monovalent salts, such as sodium salts, typically do not reduce bioadhesion as much.

Such bioadhesive polymers may be prepared by conventional free radical polymerization techniques utilizing initiators such as benzoyl peroxide, azobisisobutyronitrile, and the like. Exemplary preparations of useful bioadhesives are provided in the '697 patent.

The bioadhesive formulation may be in the form of a gel, cream, tablet, pill, capsule, suppository, film, or any other pharmaceutically acceptable form that adheres to the mucosa and does not wash away easily. The preferred formulation for the present invention is in the form of a geL

Additionally, the additives taught in the '697 patent may be mixed in with the cross-linked polymer in the formulation for maximum deseed efficacy of the delivery system or for the comfort of the patient. Such additives include, without limitation, one or more of the following: lubricants, plasticizing agents, preservatives, gel formers, tablet formers, pill formers, suppository formers, film formers, cream formers disintegrating agents, coatings, binders, vehicles, coloring agents, odor controlling agents, humectants viscosity controlling agents, pH-adjusting agents, and other similar, commonly used agents.

The present composition may be delivered to the vagina in a variety of fashions as known in the art, such as (without limitation) plunger, douche, and manually. One method of delivery is to use a device similar to those described in U.S. Design Patents Nos. D345,211 and D375,352. These devices are oblong hollow tube containers, with one end capable of being opened and the other end containing most of the composition to be delivered in a sealed container that may be used relatively easily by the patient. Said containers also maintain the formulation and treating agent in a sealed, sterile environment until use. Upon use, such a container is opened and the open end is inserted into the vagina, while the other end is squeezed to deliver the contents of the container into the vagina.

The present invention thus allows the underlying cause of the pain to be treated by delivering sufficient quantity of the treating agent to the affected tissue for an extended period of time. The delivery system provides a constant source of the drug which achieves concentrations that affect contractility of the tissue, while keeping systemic concentrations low enough to avoid adverse effects.

The anti-dysrhythmic treating agent will generally be used in its basic or unprotonated form. In this form, the local anesthetics are only slightly soluble in water. In another form, the local anesthetics may be used as water-soluble salts, such as hydrochlorides. The unprotonated form of the local anesthetic is necessary for diffusion through cellular membranes to reach the site of action. Cationic species interact preferentially with the Na⁺ channels. In a preferred embodiment, the local anesthetic is used in its basic form and is suspended in a gel or gelafying tablet for delivery.

Local anesthetics act on the uterine muscle as an antiarrhythmic and reverse uterine dyskinesia as a means of preventing pain of uterine cramping associated with dyskinesia rather than frequency of contractions. The local anesthetics also prevent endometriosis by limiting retrograde menses caused by dysrhythmic contractions, and may also aid sperm transport in women with infertility linked to mild endometriosis associated with dysmenorrhea.

Typical oral or injection forms of local anesthetics would need to achieve high blood levels in order to reach uterine tissue levels sufficient to demonstrate anti-dysrhythmic efficacy. Even so-called "trigger-point" injections would tend to cause higher blood levels, and present distinct disadvantages with regard to convenience and comfort of administration when compared to the instant formulations.

### REFERENCE EXAMPLES

The following exemplary formulations are Reference Examples. All ingredients are listed in percentage by weight.

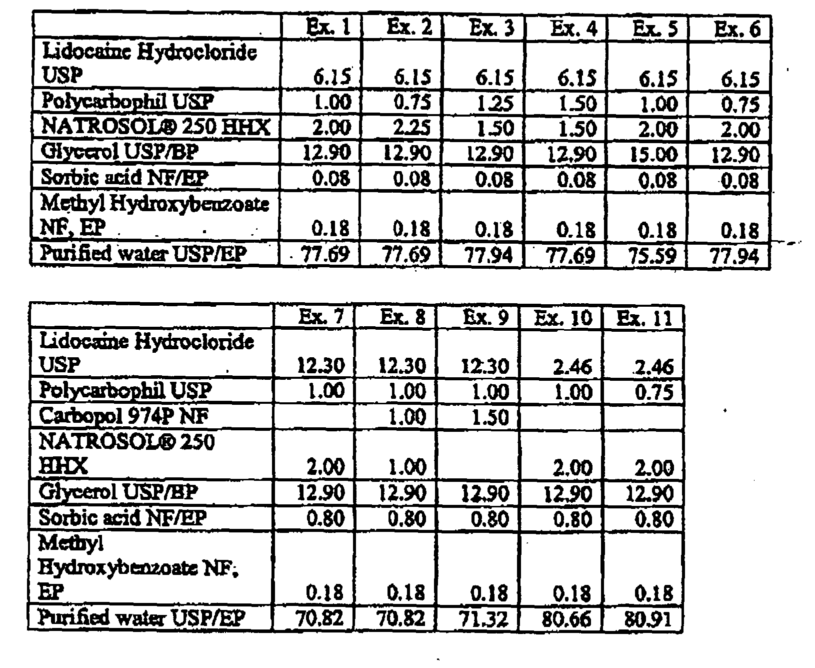

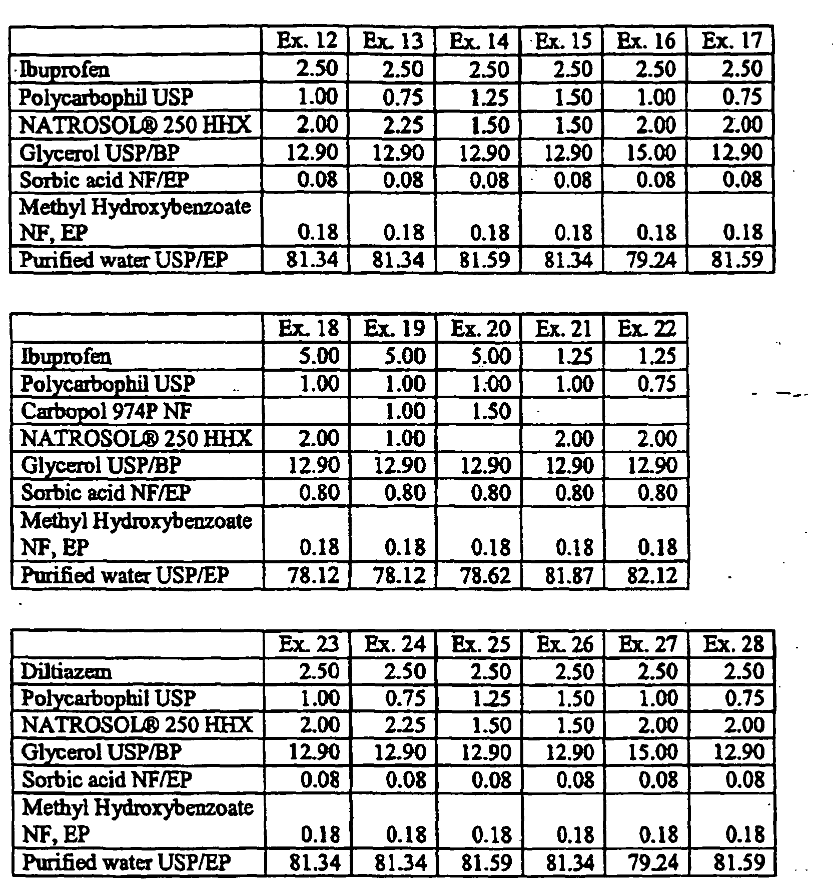

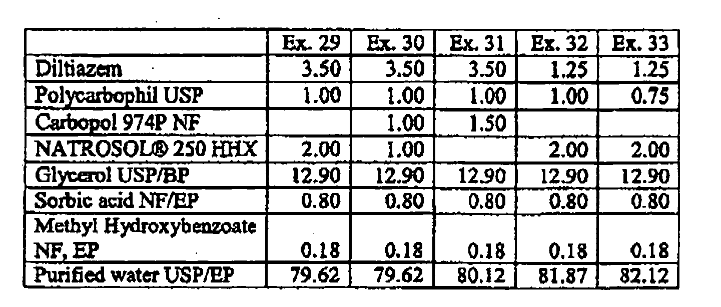

A nonlimiting example of a suitable formulation for vaginal delivery of antidysrhythmics comprises polycarbophil, carbopol, NATROSOL®, glycerol, sorbic acid, methyl hydroxybenzoate, and purified water mixed with an anti-dysrhythmic, preferably lidocaine or ibuprofen

Sorbic acid and methylhydroxybenzoate are preservatives, which may be substituted by other known preservatives, such as benzoic acid, propylparaben, or propionic acid.

Carbopol is a gel former, preferably Carbopol 974P, but may be substituted by other gel formers including, but not limited to Carbopol 934P, Carbopol 980, methyl cellulose or propyl cellulose.

NATROSOL® 250 HHX is a viscosity-enhancing agent, but may be substituted by other viscosity-enhancing agents, such as methyl cellulose or propyl cellulose.

Glycerol is a humectant; alternative humectants include, for example, "propylene glycol and dipropylene glycol

As will be apparent to those skilled in the art, the composition can be varied to affect certain properties. For example, the concentration of the bioadhesive polymer can be adjusted to provide greater or lesser bioadhesion. The viscosity can be varied by varying the pH or by changing the concentration of the polymer or gel former. The pH also can be varied as appropriate to affect the release rate or bioadhesiveness of the formulation. All ingredients are well known and readily available from supplier known in the industry, The extended-release formulations enable effective local treatment without also causing blood levels sufficient to induce adverse side effects.

Any and all publications and patent applications mentioned in this Specification are indicative of the level of skill of those skilled in the art to which this invention pertains.

## Claims

1. A pharmaceutical vaginal composition for reducing or relieving uterine dysrhythmia comprising an anti-dysrhythmic treating agent at a concentration of from 1 % to 12.5% by weight and a pharmaceutically acceptable bioadhesive carrier that releases the treating agent over an extended period of time after administration, wherein the anti-dysrhythmic treating agent is selected from prilocaine, mepivacaine, bupivacaine, ropivacaine, etidocaine, mexiletine, moricizine, propafenone, flecainide and procainamide.

2. A composition for use according to claim 1, wherein the carrier comprises a bioadhesive, water-swellable, water-insoluble, cross-linked polycarboxylic acid polymer.

3. A composition for use according to claim 2, wherein the polymer comprises polycarbophil.

4. Use of an anti-dysrhythmic treating agent and a pharmaceutically acceptable bioadhesive carrier that releases the treating agent over an extended period of time after administration, in the manufacture of a medicament for vaginal administration in reducing or relieving uterine dysrhythmia, wherein the anti-dysrhythmic treating agent is present at a concentration of from 1% to 12.5% by weight and is selected from prilocaine, mepivacaine, bupivacaine, ropivacaine, etidocaine, mexiletine, moricizine, propafenone, flecainide and procainamide.

5. Use according to claim 4, wherein the carrier comprises a bioadhesive, water-swellable, water-insoluble, cross-linked polycarboxylic acid polymer.

6. Use according to claim 5, wherein the polymer comprises polycarbophil.

## Patentansprüche

1. Vaginal verabreichbare pharmazeutische Zusammensetzung zur Verringerung oder Linderung von uteriner Dysrhythmie, umfassend ein Mittel zur Behandlung von Dysrhythmie in einer Konzentration von 1 bis 12,5 Massenanteilen und einen pharmazeutisch annehmbaren bioadhäsiven Träger, der das Behandlungsmittel nach der Verabreichung über einen längeren Zeitraum freisetzt, wobei das Mittel zur Behandlung der Dysrhythmie aus folgenden ausgewählt wird: Prilocain, Mepivacain, Bupivacain, Ropivacain, Etidocain, Mexiletin, Moricizin, Propafenon, Flecainid und Procainamid.

2. Zusammensetzung zur Venwendung gemäß Anspruch 1, wobei der Träger ein bioadhäsives, durch Wasseraufnahme quellendes, wasserunlösliches, vernetztes Polycarbonsäure-Polymer umfasst.

3. Zusammensetzung zur Verwendung gemäß Anspruch 2, wobei das Polymer Polycarbophil umfasst.

4. Verwendung eines Mittels zur Behandlung von Dysrhythmie und eines pharmazeutisch annehmbaren bioadhäsiven Trägers, der das Behandlungsmittel nach der Verabreichung über einen längeren Zeitraum freisetzt, zur Herstellung eines vaginal verabreichbaren Medikaments zur Verringerung oder Linderung von uteriner Dysrhythmie, wobei das Mittel zur Behandlung der Dysrhythmie in einer Konzentration von 1 bis 12,5 Massenanteilen vorhanden ist und aus folgenden ausgewählt wird: Prilocain, Mepivacain, Bupivacain, Ropivacain, Etidocain, Mexiletin, Moricizin, Propafenon, Flecainid und Procainamid.

5. Verwendung gemäß Anspruch 4, wobei der Träger ein bioadhäsives, durch Wasseraufnahme quellendes, wasserunlösliches, vernetztes Polycarbonsäure-Polymer umfasst.

6. Verwendung gemäß Anspruch 5, wobei das Polymer Polycarbophil umfasst.

## Revendications

1. Composition pharmaceutique vaginale destinée à réduire ou soulager la dysrythmie utérine, comprenant un agent de traitement anti-dysrythmique à une concentration de 1 % à 12,5 % en poids et un véhicule bioadhésif pharmaceutiquement acceptable qui libère l'agent de traitement sur une durée prolongée après l'administration, où l'agent de traitement anti-dysrythmique est sélectionné parmi la prilocaïne, la mépivacaïne, la bupivacaïne, la ropivacaïne, l'étidocaïne, la mexilétine, la moricizine, la propafénone, le flécaïnide et le procaïnamide.

2. Composition pour utilisation selon la revendication 1, où le véhicule comprend un polymère bioadhésif d'acide polycarboxylique réticulé, non hydrosoluble, gonflant dans l'eau.

3. Composition pour utilisation selon la revendication 2, où le polymère comprend du polycarbophile.

4. Utilisation d'un agent de traitement anti-dysrythmique et d'un véhicule bioadhésif pharmaceutiquement acceptable qui libère l'agent de traitement sur une durée prolongée après l'administration, dans la préparation d'un médicament pour administration vaginale destiné à réduire ou soulager la dysrythmie utérine, où l'agent de traitement anti-dysrythmique est présent à une concentration de 1 % à 12,5 % en poids et est sélectionné parmi la prilocaïne, la mépivacaïne, la bupivacaïne, la ropivacaïne, l'étidocaïne, la méxilétine, la moricizine, la propafénone, le flécaïnide et le procaïnamide.

5. Utilisation selon la revendication 4, où le véhicule comprend un polymère bioadhésif d'acide polycarboxylique réticulé, non hydrosoluble, gonflant dans l'eau.

6. Utilisation selon la revendication 5, où le polymère comprend du polycarbophile,
